# EUROPEAN PATENT SPECIFICATION

(11) **EP 2 086 473 B1**
(45) Date of publication and mention of the grant of the patent: **15.07.2015**
(21) Application number: 07807936.5
(22) Date of filing: 02.08.2007
(51) Int. Cl.: A61F 2/90, A61F 2/07

(54) **METHOD OF MANUFACTURING A STENT**
VERFAHREN ZUR HERSTELLUNG EINES STENTS
PROCÉDÉ DE FABRICATION D'UNE ENDOPROTHÈSE

(30) Priority: 01.11.2006 KR 20060107341
(43) Date of publication of application: 12.08.2009
(73) Proprietor: M.I. Tech Co., Ltd., Gyeonggi-do 451-864 (KR)
(72) Inventor: KIM, Seong-Hyeon, Pyeongtaek-si, Gyeonggi-do, 459-100 (KR); KIM, Young-Shin, Ansan-si, Gyeonggi-do, 425-774 (KR); KIM, Hyun-Joo, Pyeongtaek-si, Gyeonggi-do 459-110 (KR)
(74) Representative: Pulieri, Gianluca Antonio
(86) International application number: PCT/KR2007/003727
(87) International publication number: WO 2008/054054

(56) References cited:
- EP-A1- 1 550 477
- WO-A2-2005/084583
- US-A1- 2005 049 694
- US-B1- 6 375 787
- US-B1- 6 783 793
- US-B1- 6 800 089
- US-B2- 6 685 739

## Description

### [Technical Field]

The present invention relates to a method of manufacturing a stent. More particularly, the present description relates to a stent that provides a simple surgical operation for affected parts of a lumen in a human body and particularly reduces a medical side effect by contact or friction between the inside of the lumen and the outside of the stent when the stent is removed from the lumen, and the present invention relates to a method of manufacturing the stent.

### [Background Art]

In general, the main purpose of a medical stent is to expand an affected part of a lumen in a human body that has been narrowed by disease, an external injury, or an operation.

Various types of stent have been known in the art, a particularly a stent that is formed by bending wires into a zigzag pattern with peaks and valleys, forming a cylindrical structure with the peaks and valleys, and coating the cylindrical wire structure with a cover film is widely used.

The cover film prevents cancerous or fibrous tissues from permeating into the cylindrical structure through the outside of the stent that is positioned in a lumen of a human body.

The cover film is formed by inserting a cylindrical shaper into the internal passage of the wire structure and then applying a liquid coating material onto the outside of the wire structure.

According to this application, as the coating material applied on the outside of the wire structure infiltrates between the structure and the contact surface of the shaper by adhesiveness, the wires of the wire structure are embedded at the inside perimeter and exposed at the outside perimeter thereof.

Therefore, according to a stent with the cover film formed by the above application, the wires are exposed at the outside by as much as the difference in thickness between the coating layer on the wires and the coating layer, i.e. the cover film formed by only the coating material itself.

However, the stent with wires exposed at the outside causes friction due to contact between the coated wires exposed outside and the inside of the lumen when it is removed out of the lumen of the human body.

In particular, when friction is caused when removing the stent, the inside of the lumen of the human body is damaged or pain is caused. Therefore, it is difficult to ensure sufficient safety, which may be a factor that causes medical side effects in an operation using the stent.

Further, since the wires are coated but exposed at the outside of the stent, after the stent is inserted in the lumen of the human body the cellular tissues of the conduit expand into the spaces between the exposed wires by reaction, and the stent may be covered and fixed in the conduit of the lumen. Therefore, another medical side effect may be generated.

A coated stent including an extruded coating disposed on at least a portion of the stent framework is also disclosed in document US 2005/0049694 A1. Document EP 1 550 477 discloses a coated stent obtained using a rotating shaper and an UV oven.

The above information disclosed in this Background section is only for enhancement of understanding of the background of the invention and therefore it may contain information that does not form the prior art that is already known in this country to a person of ordinary skill in the art.

### [Disclosure]

### [Technical Problem]

The present invention has been made in an effort to provide a stent that allows a simple surgical operation for lumens in a human body and that particularly reduces a medical side effect caused by contact or friction between the inside of the lumen and the outside of the stent after the operation is completed, and a method of manufacturing the stent.

### [Technical Solution]

An example not forming part of the invention provides a stent including:
a cylindrical wire structure having cylindrically wound wires, each bent in a zigzag shape to have peaks and valleys; and
a cover film covering the inside and outside of the wire structure.

In the stent, the cover film is coated such that the outside of the cover film forms a circumferential surface that is the same as the coating surface on the outside of the wires of the cylindrical wire structure.

An embodiment of the present invention as disclosed in the appended claims provides a method of manufacturing a stent, including:
forming a cylindrical wire structure with an internal passage by bending wires to have peaks and valleys;
setting a shaper for coating to cover the outside of the wire structure;
coating the wire structure with a coating material while the shaper for coating having a hollow space covers the outside of the wire structure; and
forming a cover film by hardening the coating material coated on the wire structure.

### [Advantageous Effects]

As described above, according to an exemplary embodiment of the present invention, it is possible to simply manufacture a stent with a cover film that covers the inside and outside of a cylindrical wire structure that is formed by winding wires.

In particular, since the cover film that is coated on the wires arranged in a zigzag pattern in a cylindrical shape around the outside of the stent such that the wires are embedded, not exposed, in the coating material, it is possible to secure improved safety in an operation while decreasing the friction generated in removing the stent from the lumen of the human body, as compared with the stents with wires exposed at the outside in the related art.

Further, when the wires are exposed at the outside of the stent after coating, they may cause damage or pain to the inside of the lumen of the human body by friction when removing the stent from the lumen. In particular, when the cellular tissues of the lumen adhere to the wires and apply a force to the stent, the stent is depressed and the lumen may be *A* re-narrowed.

In addition, in the stent according to the present exemplary embodiment, since one end or both ends of the cylindrical wire structure are inclined by narrowing to the center, it is possible to easily remove the stent out of the lumen by preventing locking of the end(s) when removing the stent from the lumen in the human body.

### [Description of Drawings]

FIG. 1 is a flow chart illustrating preferable manufacturing processes of a method of manufacturing a stent according to an embodiment of the present invention.
FIG. 2 is a view showing a stent manufactured by the processes of FIG. 1.
FIG. 3 is a view illustrating forming a wire structure in the processes of FIG. 1.
FIG. 4 is a view illustrating forming an inclination in the processes of FIG. 1.
FIG. 5 is a view illustrating setting a shaper for coating in the processes of FIG. 1.
FIG. 6 is a view illustrating coating a coating material in the processes of FIG. 1.
FIG. 7 is a view illustrating distribution of the coating material after the coating of FIG. 6.
FIG. 8 is a view illustrating forming a cover film in the processes of FIG. 1.
FIG. 9 is a cross-sectional view of a stent manufactured through the processes of FIG. 1, which does not form part of the invention as claimed.

### [Best Mode]

Hereinafter, preferable exemplary embodiments of the invention are described in detail with reference to the accompanying drawings. The exemplary embodiments of the present invention are described such that they can be achieved by those skilled in the art.

Therefore, exemplary embodiments of the present invention can be modified into various types as long as they remain within the scope of the appended claims and the appended claims of the invention are not limited to the embodiments disclosed hereafter.

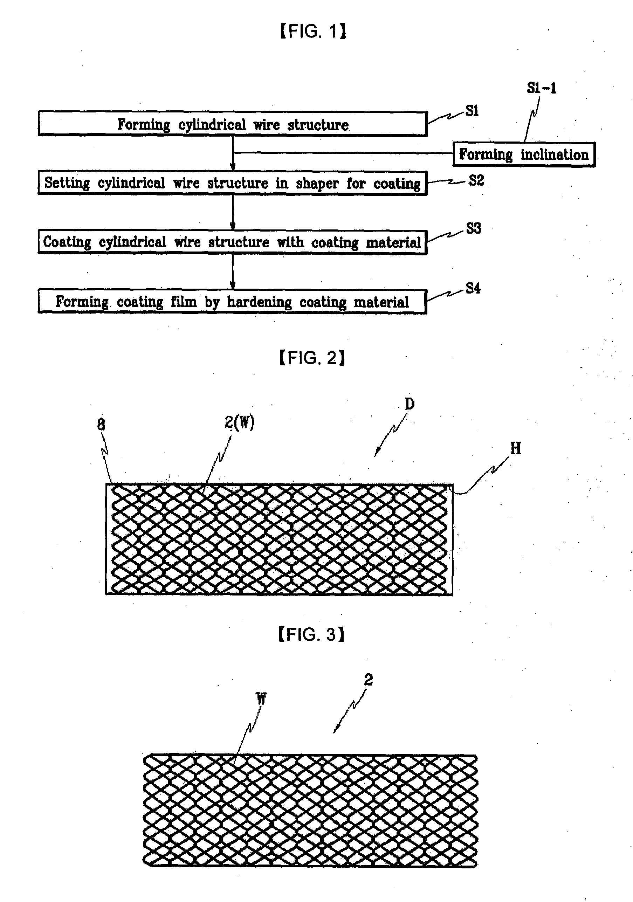

FIG. 1 is a flow chart illustrating preferable manufacturing processes of a method of manufacturing a stent according to an exemplary embodiment of the present invention, and reference numeral S1 represents forming a cylindrical wire structure.

Forming a cylindrical wire structure (S1) may be achieved by a method known in the art and is not described in detail herein.

In order to form the cylindrical wire structure, though not shown in the figures, a common jig having a bar-shaped member with a plurality of protrusions for bending that are formed around the outside for manufacturing a stent may be used.

In detail, the cylindrical wire structure 2 with the peaks and valleys is formed by bending the wires into a zigzag pattern using the jig (see FIG. 3).

According to the function required for the part of the lumen in the human body that is in need of an operation, the wire structure 2 may be, for example, a common elastic stent that elastically contracts/expands or maintains deformation.

A method of manufacturing the wire structure 2 by winding the wires W using the common jig for manufacturing a stent is widely known in the art.

Further, though not shown in the figures, the wire structure 2 may be formed in various other shapes in addition to the wires W wound as shown in FIG. 3.

The method of manufacturing a stent according to an exemplary embodiment of the present invention may further include forming an inclination (S1-1).

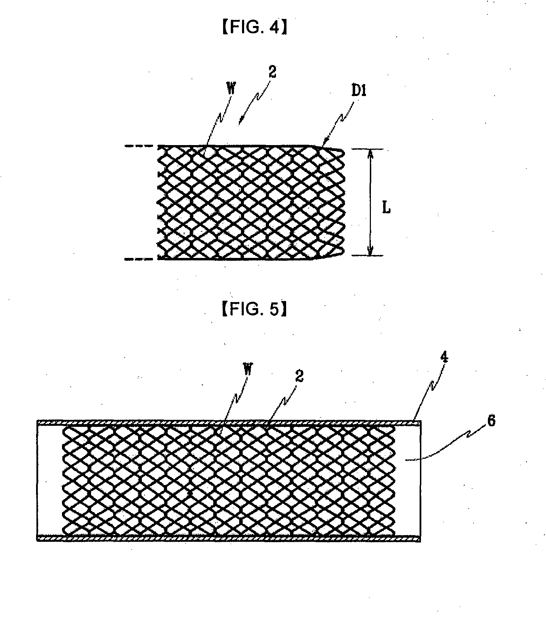

In the forming of an inclination (S1-1), when forming the wire structure 2 using a common method, that is, winding the wires W in a predetermined way using a jig for manufacturing a stent, an inclination D1 may be formed such that the wire structure 2 gradually decreases in diameter at an end by narrowing the circumference L of the end of the wire structure 2 as shown in FIG. 4,.

The inclination D1 may be formed at one end or both ends of the wire structure 2.

The wire structure 2 with the inclination D1, though not shown in the figures, has the advantage of preventing locking when removing the stent from the lumen of the human body, as compared with the wire structure of a common flare type or flat type stent in the related art.

The cylindrical wire structure 2 is set in a shaper for coating (S2).

According to the setting (S2), as shown in FIG. 5, the wire structure 2 is inserted into a hollow space 6 of the cylindrical shaper for coating 4 and set at a predetermined position.

In detail, as the wire structure 2 is inserted into the hollow space 6 of the shaper for coating 4, the entire outside of the wire structure 2 is surrounded by the inside of the hollow space 6.

The inner diameter of the hollow space 6 is the same as or less than the outer diameter of the wire structure 2, such that the wire structure 2, as shown in FIG. 5, is elastically inserted and fixed, maintaining the original shape.

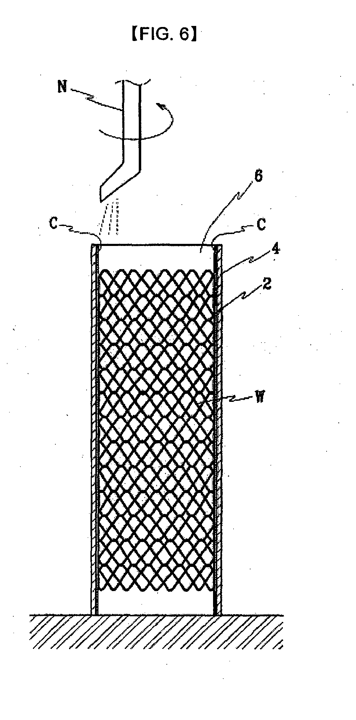

After the setting, a coating material is applied (S3). In the coating (S3), as shown in FIG. 6, a liquid coating material C may be applied from above, with the shaper for coating 4 standing as shown in FIG. 6.

The liquid coating material C may be provided to the inside of the hollow space 6 of the shaper for coating 4 using a common nozzle N. In providing the coating material, it may be possible to flow the coating material down along the entire inside of the hollow space 6 by rotating the nozzle.

It is preferable to supply as much coating material C as needed to cover the wires of the wire structure 2.

The liquid coating material C may be medical polyurethane, a silicone-urethane copolymer, silicone, a polyamide, polyester, or a fluorine resin.

As the coating is performed on the wire structure 2 covered by the shaper for coating 4 as described above, the coating material C adheres even to the inside of the hollow space 6 of the shaper for coating 4 by adhesiveness.

Since the outside of the wire structure 2 is in contact with the inside of the hollow space 6 of the shaper for coating 4, by the coating, a coating layer that forms a circumferential surface that is the same as the inside of the hollow space 6 is formed by the coating material C.

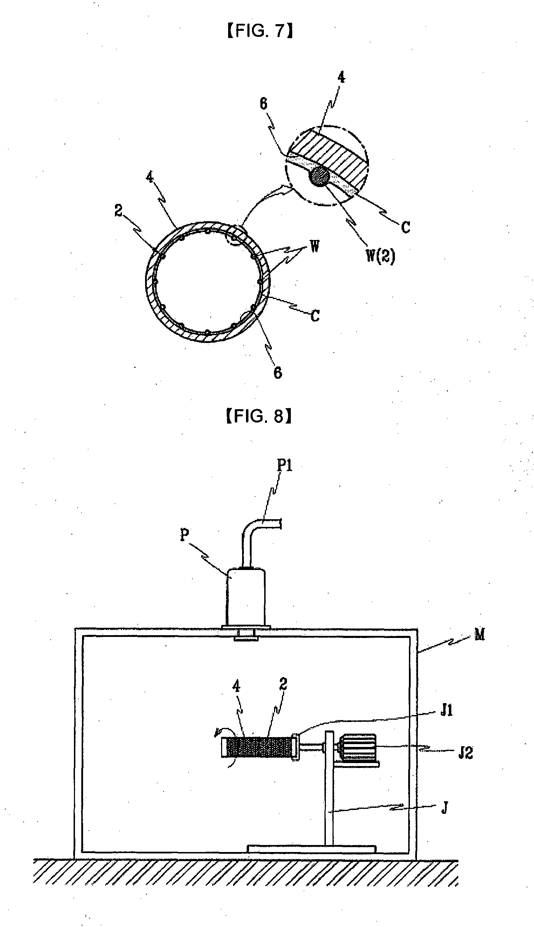

After the coating is completed, a cover film is formed (S4). The forming of the cover film (S4) is achieved by hardening the coating material C using a common UV oven M, as shown in FIG. 8.

The shaper for coating 4 with the wire structure 2 inserted therein is fixed by a holder J1 of a placing jig J.

The placing jig J is disposed in the UV oven M and holds the shaper for coating 4 horizontally.

The placing jig J may be configured such that the holder J1 that detachably holds the shaper for coating 4 is rotated by power from a motor J2.

Rotating the placing jig J with the motor is for preventing the liquid coating material C coated on the wire structure 2 from being non-uniformly distributed while flowing down.

In detail, the cover film 8 is formed, as shown in FIG. 2, by hardening the coating layer formed in a common way by the coating material C in the UV oven M while rotating the wire structure 2 that is horizontally fixed to the placing jig J.

Further, when the coating material C is hardened by the UV oven M, the hardening is achieved while removing air bubbles contained in the coating material C.

For this purpose, according the present embodiment as shown in FIG. 8, an exhaust pipe P1 connected with a common vacuum pump P communicates with the UV oven M and air bubbles contained in the coating material C are removed by vacuum pressure generated by driving the vacuum pump P.

According to the hardening under a vacuum atmosphere as described above, it is possible to easily remove the air bubbles in the coating material C coated on the wire structure 2 and prevent air bubble holes from forming on the cover film 8.

Further, since the hardening is performed while rotating the shaper for coating 4 horizontally fixed by the placing jig J under the vacuum atmosphere, it is possible to harden the infiltrated coating material C with uniformity in the space between the wires W and the contact surface of the shaper for coating 4 as well as in the space between the wires W of the wire structure 2.

Therefore, the stent D with the integral cover film 8 covering the inside and outside of the wire structure 2 as shown in FIG. 2 is manufactured by the above processes.

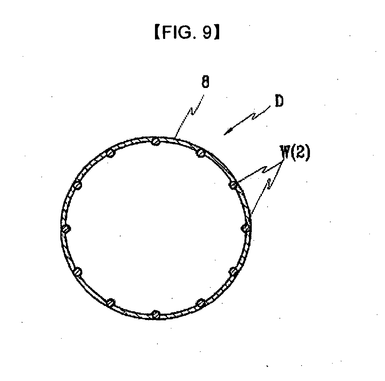

In particular, in a processe not forming part of the invention as claimed, since the coating material C is applied onto the shaper for coating 4 covering the outside of the wire structure 2, as shown in FIG. 9, it is possible to form the cover film 8 with the wires W embedded, not exposed, around the outside of the stent D by the coating material C. That is, the outside of the wire structure 2 has the cover film 8 that has the same circumferential surface as the outside of the wires W.

Further, although not shown in the drawings and not forming part of the invention as claimed, the stent D may further have a common lasso for surgical operation.

A thread or string that does not cause a side effect to the human body may be used for the lasso for surgical operation and may be connected in a way known in the art to a narrow end of the stent D by pulling.

## Claims

1. A method of manufacturing a stent by forming (S1) a cylindrical wire structure (2) with an internal passage by bending wires (H) to have peaks and valleys and coating (S3) the wire structure (2) with a coating material (C), further comprising:
setting a shaper (4) for coating to cover the outside of the wire structure (2) (S2);
coating (S3) the wire structure (2) with a coating material (C) while the shaper (4) for coating having a hollow space covers the outside of the wire structure (2); and
forming (S4) a cover film by hardening the coating material (C) coated on the wire structure (2) while rotating the wire structure (2) that is horizontally fixed to a placing jig (J) around the center;
wherein the forming of the cover film is to harden the coating material (C) under a vacuum atmosphere in the UV oven (M), using vacuum pressure formed by a vacuum pump (P).

2. The method of claim 1, further comprising
forming (S1-1) an inclination (D1) to the wire structure (2),
wherein the inclination (D1) is formed by narrowing an end of the cylindrical wire structure (2) toward the center when forming the wire structure (2).

3. The method of claim 1, wherein the setting of the shaper (4) for coating is to set the wire structure (2) in the hollow space of the shaper (4) for coating such that the outside of the wire structure (2) is covered by the inside of the hollow space.

4. The method of claim 1, wherein the forming of the cover film is to apply the coating material (C) through the hollow space of the shaper (4) for coating.

## Patentansprüche

1. Verfahren zur Herstellung eines Stents durch Ausbilden (S1) einer zylindrischen Drahtstruktur (2) mit einem Innendurchgang durch Biegen von Drähten (H), so dass sie Spitzen und Täler haben, und Beschichten (S3) der Drahtstruktur (2) mit einem Beschichtungsmaterial (C), das ferner umfasst:
Einrichten eines Formers (4) zum Beschichten, um das Äußere der Drahtstruktur (2) zu bedecken (S2);
Beschichten (S3) der Drahtstruktur (2) mit einem Beschichtungsmaterial (C), während der Former (4) zum Beschichten, der einen Hohlraum hat, das Äußere der Drahtstruktur (2) bedeckt; und
Ausbilden (S4) einer dünnen Abdeckschicht durch Härten des auf die Drahtstruktur (2) beschichteten Beschichtungsmaterials (C), während die Drahtstruktur (2), die horizontal an einer Anordnungseinspannvorrichtung (J) fixiert ist, um die Mitte gedreht wird;
wobei das Ausbilden der dünnen Abdeckschicht dazu dient, das Beschichtungsmaterial (C) unter einem Vakuumatmosphären-, UV-, Ofen (M) zu trocknen, wobei ein durch eine Vakuumpumpe (P) ausgebildeter Vakuumdruck verwendet wird.

2. Verfahren nach Anspruch 1, das ferner umfasst:
Ausbilden (S1-1) einer Neigung (D1) der Drahtstruktur (2),
wobei die Neigung (D1) ausgebildet wird, indem ein Ende der zylindrischen Drahtstruktur (2) in Richtung der Mitte verschmälert wird, wenn die Drahtstruktur (2) ausgebildet wird.

3. Verfahren nach Anspruch 1, wobei das Einrichten des Formers (4) zum Beschichten dazu dient, die Drahtstruktur (2) in dem Hohlraum des Formers (4) zum Beschichten derart einzurichten, dass das Äußere der Drahtstruktur (2) durch das Innere des Hohlraums bedeckt wird.

4. Verfahren nach Anspruch 1, wobei das Ausbilden der dünnen Abdeckschicht dazu dient, das Beschichtungsmaterial (C) durch den Hohlraum des Formers (4) zum Beschichten aufzutragen.

## Revendications

1. Procédé de fabrication d'une endoprothèse par formation (S1) d'une structure en fil métallique cylindrique (2) ayant un passage interne par courbure des fils métalliques (H) pour obtenir des pointes et des creux et revêtement (S3) de la structure en fil métallique (2) avec un matériau de revêtement (C), comprenant en outre :
la mise en place d'un dispositif de mise en forme (4) destiné au revêtement pour recouvrir l'extérieur de la structure en fil métallique (2) (S2) ;
le revêtement (S3) de la structure en fil métallique (2) avec un matériau de revêtement (C) pendant que le dispositif de mise en forme (4) destiné au revêtement ayant un espace creux recouvre l'extérieur de la structure en fil métallique (2) ; et
la formation (S4) d'un film de revêtement par durcissement du matériau de revêtement (C) enrobé sur la structure en fil métallique (2) pendant la rotation de la structure en fil métallique (2) qui est fixée horizontalement à un gabarit de pose (J) autour du centre ;
dans lequel la formation du film de revêtement consiste à durcir le matériau de revêtement (C) sous une atmosphère de vide dans le four à ultraviolet (UV) (M), en utilisant la pression sous vide générée par une pompe à vide (P).

2. Procédé selon la revendication 1, comprenant en outre :
la formation (S1-1) d'une inclinaison (D1) de la structure en fil métallique (2),
dans lequel l'inclinaison (D1) est formée par rétrécissement d'une extrémité de la structure en fil métallique cylindrique (2) vers le centre lors de la formation de la structure en fil métallique (2).

3. Procédé selon la revendication 1, dans lequel la mise en place du dispositif de mise en forme (4) destiné au revêtement consiste à mettre en place la structure en fil métallique (2) dans l'espace creux du dispositif de mise en forme (4) destiné au revêtement de telle sorte que l'extérieur de la structure en fil métallique (2) soit recouvert par l'intérieur de l'espace creux.

4. Procédé selon la revendication 1, dans lequel la formation du film de revêtement consiste à appliquer le matériau de revêtement (C) à travers l'espace creux du dispositif de mise en forme (4) destiné au revêtement.
